# EUROPÄISCHE PATENTSCHRIFT

(11) **EP 1 027 452 B1**
(45) Veröffentlichungstag und Bekanntmachung des Hinweises auf die Patenterteilung: **06.03.2002**
(21) Anmeldenummer: 98952742.9
(22) Anmeldetag: 24.10.1998
(51) Int. Cl.: C12P 19/24, C12N 11/02, C13K 13/00, C12N 9/90, A23L 1/236

(54) **VERFAHREN ZUR HERSTELLUNG VON ISOMELEZITOSE UND ISOMELEZITOSE-HALTIGEN SÜSSUNGSMITTELN**
METHOD FOR PRODUCING ISOMELEZITOSE AND SWEETENING AGENTS CONTAINING ISOMELEZITOSE
PROCEDE POUR LA PREPARATION D'ISOMELEZITOSE ET D'EDULCORANTS EN CONTENANT

(30) Priorität: 29.10.1997 DE 19747642
(43) Veröffentlichungstag der Anmeldung: 16.08.2000
(73) Patentinhaber: SÜDZUCKER AKTIENGESELLSCHAFT MANNHEIM/OCHSENFURT, 68165 Mannheim (DE)
(72) Erfinder: MUNIR, Mohammad, D-67271 Kindenheim (DE); VOGEL, Manfred, D-67271 Neuleiningen (DE)
(74) Vertreter: Gleiss & Grosse
(86) Internationale Anmeldenummer: EP9806768
(87) Internationale Veröffentlichungsnummer: WO9922013

(56) Entgegenhaltungen:
- EP-A- 0 091 063
- EP-A- 0 625 578
- EP-A- 0 794 259
- DE-B- 2 217 628
- PATENT ABSTRACTS OF JAPAN vol. 017, no. 678 (C-1141), 13. Dezember 1993 & JP 05 227955 A (SUNTORY LTD), 7. September 1993 in der Anmeldung erwähnt
- CHEMICAL ABSTRACTS, vol. 105, no. 25, 22. Dezember 1986 Columbus, Ohio, US; abstract no. 222353, FUJII, SATOSHI ET AL: "Oligosaccharides produced by the transglucosidation action of Protaminobacter rubrum.alpha.-glucosidase" XP002092972 in der Anmeldung erwähnt & SEITO GIJUTSU KENKYU KAISHI (1985), (34), 37-44 CODEN: SGIKA6;ISSN: 0370-9841, 1985,
- DATABASE FSTA INTERNATIONAL FOOD INFORMATION SERVICE (IFIS), FRANFURT/MAIN, DE FUJII S ET AL: "Isolation and characterization of oligosaccharides produced from sucrose by transglycosylation action of Serratia plymuthica." XP002092973 & JOURNAL OF JAPANESE SOCIETY OF FOOD SCIENCE AND TECHNOLOGY ((NIPPON SHOKUHIN KOGYO GAKKAISHI)), Bd. 30, Nr. 6, 1983, Seiten 339-344,

## Beschreibung

Die vorliegende Erfindung betrifft ein Verfahren zur Herstellung von Süßungsmitteln, insbesondere Isomelezitose-haltigen Süßungsmitteln, reiner Isomelezitose sowie die mittels dieser Verfahren hergestellten Süßungsmittel.

Isomelezitose, ein Trisaccharid, weist eine Reihe interessanter Eigenschaften auf. Insbesondere wird Isomelezitose nicht durch Speichelenzyme oder im Mundraum vorhandenen Bakterien gespalten. Isomelezitose weist ferner eine gute pH-Stabilität auf und wird auch im Dünndarm nicht gespalten. Schließlich ist Isomelezitose ein Substrat für nützliche bifidogene Mikroorganismen im Dickdarm. Aufgrund dieser Eigenschaften führt die Verwendung von Isomelezitose in Nahrungsmitteln zu einer Brennwertminderung, einer Zahnschonung, sowie zur Ausbildung einer günstigen Mikroorganismenpopulation im Dickdarm. Zudem ist Isomelezitose diabetikergeeignet.

Die Herstellung von Isomelezitose ist beispielsweise von Chiba et al. (Agric. Biol. Chem. 43 (4) (1979), 775 bis 779) beschrieben. Diese Druckschrift offenbart die Herstellung von Isomelezitose, wobei eine Saccharose-Lösung bei 30°C fünf Stunden lang mit einer α-Glucosidase aus Hefe inkubiert und eine Isomelezitose-haltige Lösung erhalten wird. In den japanischen Offenlegungsschriften JP 0430796 und JP 05227955 sowie JP 0430771 wird die Herstellung von Isomelezitose zusammen mit Theanderose und Isomelezitose-, Theanderosehaltigen Nahrungsmitteln unter Verwendung eines thermostabilen Enzyms aus Bacillus sp. bei 65°C beschrieben. Fujii et al. (Seito Gijutsu Kenkyu Kaishi 34 (1985), 37 bis 44) beschreibt die Herstellung von Isomelezitose unter Verwendung immobilisierter oder freier Zellen von Protaminobacter rubrum bei 30°C und einer Inkubationszeit von 48 Stunden.

Die EP 0 625 578 A1 beschriebt die Herstellung eines nicht-kariogenen und kalorienarmen Süßungsmittels unter Einsatz enzymatischer Umlagerungsreaktionen von Saccharose mit anschliessender katalytischer Hydrierung der Umlagerungsprodukte, wobei ein geringer Gehalt an Oligosacchariden entsteht.

Die beschriebenen Verfahren sind jedoch im Hinblick auf eine effiziente Herstellung von Isomelezitose in hoher Reinheit und Ausbeute verbesserungswürdig. Zudem führen die Verfahren nicht zu Süßungsmitteln, die sich durch Diabetikereignung, Akariogenität sowie vorzugsweise einen günstigen Einfluß auf die Mikroorganismenpopulation im menschlichen Verdauungstrakt auszeichnen.

Das der vorliegenden Erfindung zugrundeliegende technische Problem besteht also in der Bereitstellung eines verbesserten Verfahrens zur Herstellung von Isomelezitose sowie eines Verfahrens zur Herstellung von Süßungsmitteln.

Die Erfindung löst das technische Problem durch die Bereitstellung eines Verfahrens zur Herstellung eines Süßungsmittels, wobei man in einem ersten Verfahrensschritt Saccharose enzymatisch in ein Saccharidgemisch bei einer Temperatur von 50 bis 60°C umwandelt und in einem zweiten Verfahrensschritt das erhaltene Saccharidgemisch katalytisch hydriert. Je nachdem, ob man aus dem erhaltenen Saccharidgemisch Isomelezitose abtrennt oder nicht, erhält man nach Hydrierung des Saccharidgemisches ein Süßungsmittelgemisch, das entweder Isomelezitose in vergleichsweise großer Menge oder gar keine Isomelezitose enthält.

Die Erfindung löst das technische Problem auch durch die Bereitstellung eines Verfahrens zur Herstellung von Isomelezitose und Isomaltulose, wobei man Saccharoselösung enzymatisch in eine Lösung eines Saccharidgemisches bei einer Temperatur von 50 bis 60°C umwandelt, aus der so erhaltenen Lösung eines Saccharidgemisches die entstandene Isomaltulose durch Kristallisation, vorzugsweise Verdampfungs- und Kühlungskristallisation, weitgehend entfernt und dabei die Isomelezitose in einer Mutterlauge anreichert, die Mutterlauge einer chromatographischen Trennung unterwirft, dabei aus dem Eluat die an Isomelezitose angereicherten Fraktionen gezielt sammelt und diese zu einer Isomelezitose-Fraktion vereinigt, die in einem weiteren Verfahrensschritt weiter aufkonzentriert, insbesondere eingedampft und kühlungskristallisiert, zentrifugiert und gewaschen wird, wobei Isomelezitose in reiner kristalliner Form erhalten wird. Die erhaltene Isomaltulose kann entweder in dieser Form weiter verwendet werden oder erfindungsgemäß katalytisch zu einem als hydrierte Isomaltulose bezeichneten Süßungsmittel hydriert werden.

Die Erfindung zeichnet sich also dadurch aus, daß man Saccharose, insbesondere eine wäßrige Saccharose-Lösung, bei einer Temperatur von 50 bis 60°C enzymatisch, vorzugsweise unter Verwendung von Protaminobacter rubrum, insbesondere Protaminobacter rubrum CBS 574.77, in ein Saccharidgemisch beziehungsweise eine wäßrige Lösung eines Saccharidgemisches umwandelt. Die enzymatische Umlagerung der Saccharose führt zu einem in Zusammenhang mit der vorliegenden Erfindung als Saccharidgemisch bezeichneten Gemisch aus Fructose, Glucose, Isomaltulose, Trehalulose, Isomaltose, Isomelezitose und Spuren an anderen Trisacchariden sowie Restsaccharose. Dieses Saccharidgemisch weist überraschenderweise aufgrund der bei 50 bis 60°C erfolgenden Umlagerung einen gegenüber einer Umlagerung bei üblichen 30°C (0,4 Gew.-% auf Trockensubstanz (a.TS)) stark erhöhten Isomelezitose-Gehalt von über 10% Gew.-% auf Trockensubstanz auf. Aus diesem Saccharidgemisch können einzelne Substanzen je nach Anwendungszweck abgetrennt, beispielsweise durch Kristallisation oder Chromatographie, werden, beispielsweise Isomelezitose und/oder Trehalulose.

In vorteilhafter Weise sieht die Erfindung dabei vor, die enzymatische Umwandlung mittels freier oder immobilisierter Zellen von Protaminobacter rubrum CBS 574.77 oder mittels des isolierten Enzyms, das heißt der Saccharose-6-Glucosylmutase (EC 5.4.99.10), durchzuführen. Die Anzucht und Immobilisierung des Bakterienstammes Protaminobacter rubrum (CBS 574.77) erfolgt nach bekannten Methoden, zum Beispiel wie in der EP 0 028 900, DE 30 38 219 oder EP 0 625 578 beschrieben.

In besonders vorteilhafter Weise sieht die Erfindung vor, daß die, vorzugsweise immobilisierten, Zellen von Protaminobacter rubrum verbraucht sind, das heißt, vor Einsatz im erfindungsgemäßen Verfahren bereits für eine enzymatische Umwandlung von Saccharose zu im wesentlichen Isomaltulose eingesetzt wurden. Im Zusammenhang mit der vorliegenden Erfindung werden unter verbrauchten, vorzugsweise immobilisierten, Zellen solche Protaminobacter rubrum-Zellen verstanden, deren Aktivität während einer vorangegangenen, durch diese Zellen bewirkten herkömmlichen Umwandlung von Saccharose zu im wesentlichen Isomaltulose bei 30°C so weit reduziert wurde, daß die Zellen für diesen Zweck, das heißt die herkömmliche Umwandlung von Saccharose zu im wesentlichen Isomaltulose nicht mehr wirtschaftlich eingesetzt werden können.

In einer bevorzugten Ausführungsform der vorliegenden Erfindung wurden die vorzugsweise immobilisierten, Zellen vor ihrem Einsatz als verbrauchte Zellen im erfindungsgemäßen Verfahren mindestens 100 Tage, vorzugsweise mindestens 150 Tage, besonders bevorzugt 160 Tage, für eine enzymatische Umwandlung von Saccharose zu im wesentlichen Isomaltulose bei 30°C oder unter im wesentlichen gleichwertigen Bedingungen eingesetzt, wobei 40 bis 45 Gew.-% Saccharose (a. TS) in wäßriger Lösung aus Ausgangslösung verwendet wurden.

In einer besonders bevorzugten Ausführungsform der vorliegenden Erfindung weisen verbrauchte, vorzugsweise immobilisierte, Protaminobacter rubrum-Zellen eine Enzymaktivität von maximal 200 bis 250 Einheiten/pro g Trockensubstanz (TS) Biokatalysator auf (1 Einheit entspricht 1 µmol umgesetzte Saccharose pro Minute). In bevorzugter Ausführungsform der vorliegenden Erfindung sind die verbrauchten, eine Aktivität von maximal 200 bis 250 Einheiten/pro g Biokatalysator (TS) aufweisenden, vorzugsweise immobilisierten, Zellen aus frischen, unverbrauchten, vorzugsweise immobilisierten, Zellen durch Einsatz in einer Umwandlungsreaktion von Saccharose zu im wesentlichen Isomaltulose gewonnen worden, wobei die frischen Zellen ursprünglich eine Aktivität von mindestens 400 bis 450 Einheiten/g Trockensubstanz (TS) Biokatalysator aufwiesen.

Die erfindungsgemäß in bevorzugter Weise einzusetzenden verbrauchten, vorzugsweise immobilisierten, Protaminobacter rubrum Zellen zeichnen sich beispielsweise in vorteilhafter Weise dadurch aus, daß sie gegenüber nicht verbrauchten, frischen Zellen eine erheblich verlängerte Benutzungsdauer ermöglichem. Zudem führt ihr Einsatz zu einem größeren Anteil an Isomelezitose im Umlagerungsprodukt. Schließlich ist die erfindungsgemäße Vorgehensweise insofern, als daß verbrauchte, an sich kommerziell nicht mehr einsetzbare Zellen, also Biomüll, einem neuen vorteilhaften Verwendungszweck zugeführt werden können und demgemäß nicht in kostenintensiver und arbeitsintensiver Weise entsorgt werden müssen.

Überraschenderweise sind die erfindungsgemäß einzusetzenden verbrauchten, vorzugsweise immobilisierten, Zellen von Protaminobacter rubrum in der Lage, ein Isomaltulose-Isomelezitose-Gemisch mit einem besonders hohen Isomelezitose-Anteil zu bilden.

Die Erfindung sieht in vorteilhafter Weise weiterhin vor, daß die enzymatische Umlagerung, vorzugsweise kontinuierlich über einen Zeitraum durchgeführt wird, der ausreicht, die Saccharose im wesentlichen vollständig umzusetzen. Die Umlagerung kann kontinuierlich auch über einen Zeitraum von 40 bis 50 Tagen durchgeführt werden.

Die Erfindung sieht in einer weiteren Ausführungsform vor, mit dem Saccharidgemisch, das entweder Isomelezitose in großer Menge, d.h. mehr als 10 Gew.-% (a.TS), oder nach Abtrennung von Isomelezitose, wenig oder gar keine Isomelezitose mehr enthält, in vorteilhafter Weise eine weitere enzymatische Umlagerung unter Verwendung des vorgenannten Enzyms oder des vorgenannten Bakteriums durchzuführen, vorzugsweise bei einer Temperatur von 25 bis 30°C. Diese zweite enzymatische Umlagerung führt zu einer vollständigen Umwandlung möglicherweise nach der ersten Umlagerung noch vorhandener Restsaccharose im Saccharidgemisch bei gleichzeitig geringer Zunahme an Isomelezitose.

Im Anschluß an die erste oder zweite enzymatische Umlagerung kann das entstandene Saccharidgemisch zur Herstellung reiner Isomelezitose und Isomaltulose verwendet werden. Nach Aufkonzentrierung des Saccharidgemisches, insbesondere mittels Verdampfungs- und/oder Kühlungskristallisation, anschließender Abtrennung von Isomaltulose und Durchlaufens der gebildeten Mutterlauge eines Chromatographiesowie weiterer Aufkonzentrierungs-, insbesondere Kühlungskonzentrationsschritte, wird reine Isomelezitose erhalten. Diese Vorgehensweise führt zu der Herstellung von Isomaltulose und kristalliner Isomelezitose. Erfindungsgemäß kann vorgesehen sein, die isolierte Isomaltulose in einem weiteren Verfahrensschritt zu hydrierter Isomaltulose, vorzugsweise mittels eines Raney-Nickel-Katalysators, zu hydrieren.

Die Erfindung sieht jedoch auch vor, daß das aus der ersten oder zweiten Umlagerung hervorgehende Saccharidgemisch eventuell nach Abtrennung einzelner Komponenten wie Isomelezitose oder Trehalulose hydriert wird. Die Hydrierung wird vorzugsweise an einem Raney-Nickel-Katalysator mit Wasserstoffgas in an sich bekannter Weise durchgeführt. Die Hydrierung führt zu einem Süßungsmittelgemisch, das 1,1-GPM (1-O-α-D-Glucopyranosyl-D-mannit), 1,6-GPS (6-O-α-D-Glucopyranosyl-D-sorbit) sowie, falls nicht vorher abgetrennt, Isomelezitose und gegebenenfalls Resttrisaccharide, Mannit, Sorbit, 1,1-GPS (1-O-α-D-Glucopyranosyl-D-sorbit) enthält. Ein derartiges erfindungsgemäßes Süßungsmittelgemisch mit einem erhöhten Isomelezitose-Gehalt von über 10%-Gew.-%, vorzugsweise von 10 bis 18 Gew.-% (bezogen auf TS), ist vorteilhaft insofern, als daß dieses Süßungsmittelgemisch brennwertreduziert, zahnschonend und prebiotisch ist.

Selbstverständlich ist es auch möglich, das nach der ersten oder zweiten Umlagerung gewonnene Saccharidgemisch chromatographischen Trennschritten zu unterziehen, die beispielsweise zur Abtrennung von Trehalulose führen können. In einem solchen Fall enthält das Süßungsmittelgemisch nach Hydrierung die vorgenannten Bestandteile, nicht jedoch 1,1-GPS. Das enthaltene Süßungsmittelgemisch kann dann als Süßungsmittelgemisch aus mehr als 10 Gew.-% Isomelezitose und Isomalt® (hydrierte Isomaltulose, Palatinit®) angesehen werden. Die Erfindung sieht jedoch auch vor, daß Isomelezitose aus dem Saccharidgemisch abgetrennt wird. In einem solchen Fall wird, je nach dem, ob Trehalulose ebenfalls abgetrennt wurde, entweder ein Gemisch aus 1,1-GPM, 1,6-GPS, Mannit und Sorbit oder aus 1,1-GPS, 1,1-GPM, 1,6-GPS, Mannit und Sorbit hergestellt.

Die Erfindung sieht auch vor, daß das aus der ersten oder zweiten enzymatischen Umlagerung hervorgehende Saccharidgemisch vor der Hydrierung einer Behandlung mit Bäckerhefe unterzogen wird. Insbesondere kann gemäß einer besonders bevorzugten Ausführungsform der Erfindung vorgesehen sein, das erhaltene Saccharidgemisch mit freien oder immobilisierten Zellen von Bäckerhefe in Kontakt zu bringen, wobei die Verweilzeit so gewählt wird, daß eventuell vorhandene Monosaccharide, zum Beispiel Glucose und Fructose, vergoren, insbesondere vollständig vergoren, werden können.

Im Anschluß an die Hydrierung kann erfindungsgemäß vorgesehen sein, die aus der Hydrierung hervorgehende Süßungsmittelgemisch-Lösung zu trocknen, so daß Süßungsmittel in trockener Form erhalten wird.

Die enzymatische Umwandlung oder Isomerisierung von Saccharose zu Isomaltulose mittels immobilierter Zellen von Protaminobacter rubrum (CBS 574.77) erfolgt herkömmlicherweise in technischem Maßstab in einem temperierbaren Säulenreaktor bei Temperaturen von 25 - 30°C. Ausgehend von einer Saccharose-Lösung mit einem TS-Gehalt von 40% wird die Fließgeschwindigkeit so eingestellt, daß mindestens 97% der eingesetzten Saccharose umgelagert werden.

Eine HPLC-Analyse der umgelagerten Saccharose-Lösung hat zum Beispiel folgende Zusammensetzung:

**Tabelle 1**

| | % a.TS |
|---|---|
| Fructose | 2,5 |
| Glucose | 2,0 |
| Saccharose | 1,0 |
| Isomaltose | 1,2 |
| Isomaltulose | 82,5 |
| Trehalulose | 9,5 |
| Isomelezitose | 0,4 |
| Trisaccharide | 0,5 |

Erfindungsgemäß wurde nun überraschenderweise gefunden, daß bei Umlagerungstemperaturen von 50 bis 62°C, insbesondere 50 bis 60°C, besonders bevorzugt 50-58°C die Gehalte an Isomelezitose über 10 Gew.-%, vorzugsweise zwischen 11 und 18 Gew.-% a.TS, betragen. Des weiteren erweist sich das für die Umwandlung eingesetzte Enzym, die Saccharose-6-glucosylmutase (EC 5.4.99.10) in den immobilisierten Zellen des erfindungsgemäß eingesetzten mesophilen Bakterienstammes Protaminobacter rubrum (CBS 574.77) als so stabil, daß bis zum Erreichen der halb-maximalen Leistung Umlagerungen für 40 bis 50 Tage, insbesondere 45 Tage, bei 50°C durchgeführt werden können, wobei über die Kontaktzeit an Immobilisat Gehalte an Isomelezitose von ≥ 10% a.TS und Gehalte an Disacchariden von vorzugsweise mehr als 80 Gew.% a.TS erhalten werden.

Ausgehend von dieser Isomelezitose-reichen Umlagerungslösung, also dem Saccharidgemisch, mit einem mittleren Gehalt von 11 Gew.-% a.TS an Isomelezitose ist es erfindungsgemäß möglich, mit wenigen Isolierungsschritten zu einer kristallinen Isomelezitose von hoher Reinheit zu gelangen, wobei gleichzeitig Isomaltulose gewonnen wird. Diese Vorgehensweise ist noch vorteilhafter und wirtschaftlicher durchzuführen, wenn die erhaltene Isomelezitose-reiche Umlagerungslösung einer weiteren Behandlung in vorzugsweise einem Säulenreaktor, gefüllt mit immobilisierten Protaminobacter-Zellen, bei 25 bis 30°C unterzogen wird. In dieser Ausführungsform läßt sich verbliebene Saccharose besonders mühelos vollständig isomerisieren.

Wird eine vollständig isomerisierte Saccharoselösung, also das Saccharidgemisch, mit 10 bis 12 Gew.-% Isomelezitose als Rohstoff eingesetzt, kann die Isomelezitose- und Isomaltulosegewinnung folgendermaßen durchgeführt werden. Zunächst wird das Saccharidgemisch auf 75 bis 85% (%-Angaben sind im folgenden Gew.-%, falls nicht anders angegeben) a.TS aufkonzentriert. Eine sich anschließende Kühlungskristallisation mit einer Kühlungsrate von 1 bis 2°K pro Stunde wird zwischen 80 bis 85°C gestartet und erfolgt unter langsamem Rühren bis zu einer Temperatur von 40 bis 50°C. Die erhaltenen Kristalle lassen sich mit einer geeigneten Zentrifuge, zum Beispiel einer Siebkorbzentrifuge, von der Mutterlauge abtrennen. Nach Waschen der Kristalle mit etwas Wasser werden weiße Kristalle an Isomaltulose mit einer Reinheit von 97 bis 99% erhalten. Die Ausbeute -bezogen auf eingesetzte Isomaltulose- beträgt zwischen 55 bis 65%. Die kristalline Isomaltulose kann als solche direkt verwendet werden beziehungsweise erfindungsgemäß weiter zu Isomalt® mittels beispielsweise Raney-Nikkel und Wasserstoffgas hydriert werden. Die erhaltene Mutterlauge weist Gehalte an Isomelezitose zwischen 23 und 26% a.TS auf und kann als Isomelezitose-reiche Umlagerungslösung einer weiteren Verarbeitung zugeführt werden oder für die weitere Aufreinigung der Isomelezitose dienen.

Die Chromatographie der auf 40 bis 60% a.TS eingeengten Lösung erfolgt vorzugsweise an einem stark sauren schwach vernetztem Kationenaustauscherharz in der Calcium-Form Dei einer Temperatur von 60 bis 80°C mit entionisiertem Wasser. Die Isomelezitose eluiert zuerst, gefolgt von Isomaltulose, Isomaltose und Trehalulose sowie Glucose und Fructose, wobei letztere mit hoher Reinheit abgetrennt wird. Fraktionen mit Gehalten an Isomelezitose von > 63% a.TS werden vereint und auf 60 bis 75% a.TS konzentriert.

Mittels Kühlungskristallisation läßt sich aus dieser aufkonzentrierten Lösung die Isomelezitose kristallin darstellen. Je nach eingesetzter Reinheit und TS-Gehalt der Lösung beträgt die Temperatur zu Beginn der Kühlungskristallisation zwischen 50 und 75°C und die Endtemperatur 15 bis 35°C. Bei einer Abkühlungsrate von 1 bis 2°K pro Stunde lassen sich zwischen 50 und 65% der eingesetzten Isomelezitose als Kristalle durch Zentrifugation gewinnen. Die Reinheiten der gewaschenen Kristalle betragen zwischen 97 und 99% a.TS. Eine weitere Kühlungskristallisation ist entsprechend der Reinheit an Isomelezitose möglich, um so eine Steigerung an Ausbeute zu erzielen. Bezogen auf die anfänglich in dem Saccharidgemisch vorhandene Isomelezitose werden Ausbeuten von 50 bis 68% a.TS erhalten.

Zur Herstellung der erfindungsgemäßen Süßungsmittel aus 1,1-GPM, 1,6-GPS und gegebenenfalls Isomelezitose, Mannit, Sorbit und 1,1-GPS werden die vorstehend beschriebenen Saccharidgemische, gegebenenfalls nach Abtrennung von Isomelezitose und/oder Trehalulose katalytisch hydriert. Die zu hydrierenden Saccharidgemische können aus der ersten Umlagerungsreaktion, der zweiten Umlagerungsreaktion oder aus im Anschluß an die Umlagerung folgenden Konzentrierungs- und Reinigungsschritten hervorgehen.

Isomelezitose wird von Bäckerhefe nicht vergoren beziehungsweise verstoffwechselt, so daß die angeführten Isomelezitose-reichen oder gegebenenfalls nach Abtrennung von Isomelezitose und/oder Trehalulose erhaltenen Umlagerungslösungen durch Inkubation mit Bäckerhefe vor der Hydrierung zu einer Glucose-Fructose-freien Produktlösung führen. Noch verbliebene Spuren an Saccharose lassen sich auf diese Weise ebenfalls entfernen. Je nach gewünschter Fermentationszeit sind die Mengen an Bäckerhefe zu dosieren, wobei es gleichgültig ist, ob frische. Bäckerhefe oder Trockenhefe eingesetzt wird. Die erhaltene Glucose-Fructose-freie Lösung kann entweder hydriert werden oder zur Isomelezitose-Herstellung eingesetzt werden.

Sowohl die mit als auch die ohne Bäckerhefe behandelte, vorzugsweise Isomelezitose-reiche, Umlagerungslösung wird nun nach einer der an sich bekannten Methoden hydriert. Die erhaltenen, hydrierten Gemische bestehen im Falle einer vorgeschalteten Hefefermentation praktisch nur aus 1,1-GPM, 1,6-GPS und Isomelezitose beziehungsweise weisen ohne Hefefermentation noch zusätzlich Sorbit und Mannit auf. Falls Trehalulose nicht entfernt wurde, enthält das erfindungsgemäße Süßungsmittelgemisch zusätzlich 1,1-GPS. Die Erfindung sieht jedoch auch die Bereitstellung von Süßungsmittelgemischen aus 1,1-GPM und 1,6-GPS vor, die nach Umlagerung, Isomaltulose-Abtrennung und Isomaltulose-Hydrierung erhalten werden.

Ferner sieht die Erfindung die Bereitstellung von Süßungsmitteln aus 1,1-GPM, 1,6-GPS und gegebenenfalls 1,1-GPS sowie Mannit und Sorbit vor, die nach Umlagerung, Isomelezitose-Abtrennung, gegebenenfalls Trehalulose-Abtrennung und Hydrierung des erhaltenen Saccharidgemisch erhalten werden.

Selbstverständlich kann auch eine enzymatische Umlagerung einer Mischung von Mutterlauge aus einer Isomalt®-Produktionsanlage und zugesetzter Saccharose bei 50°C vorgesehen sein.

Weitere vorteilhafte Ausgestaltungen der Erfindung ergeben sich aus den Unteransprüchen.

Die Erfindung wird anhand einer Figur und dazugehöriger Ausführungsbeispiele näher erläutert.

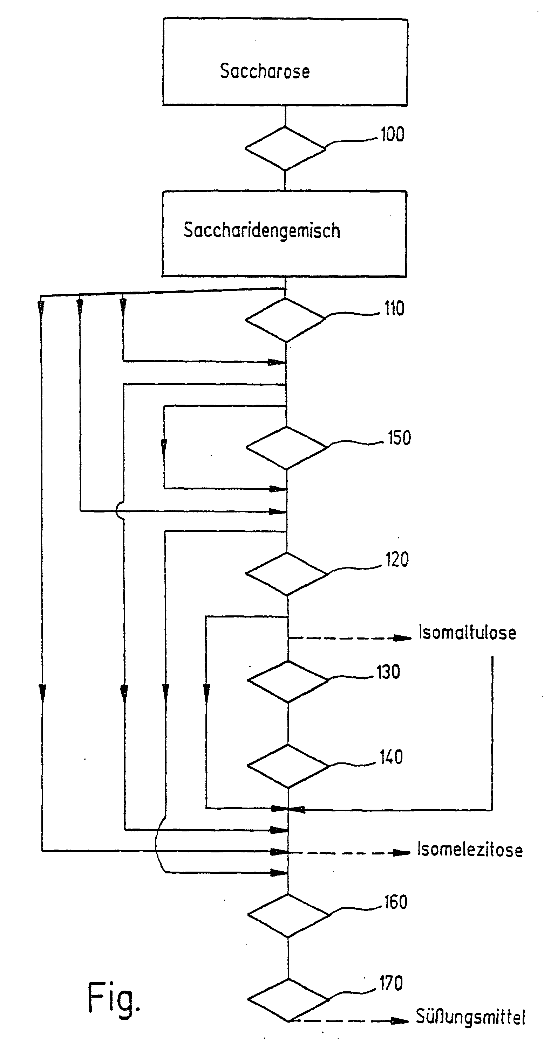

Die Figur stellt ein Fließschema der erfindungsgemäßen Verfahren dar.

### Beispiel 1:

### Herstellung eines Saccharidgemisches/Einfluß der Temperatur auf dessen Zusammensetzung

27 g getrocknete und verbrauchte immobilisierte Zellen von Protaminobacter rubrum (CBS 574.77) (200 Einheiten/g TS (Trockensubstanz) Biokatalysator) werden zunächst in einer Saccharose-Lösung mit 40% TS-Gehalt vorgequollen und anschließend in einen temperierbaren Säulenreaktor gefüllt (Schritt 100, Figur). Die Fließrate wird so gewählt, daß der Rest-Saccharose-Gehalt bei einer Arbeitstemperatur von 28°C um 1% a.TS beträgt. Als Reaktorzulauf wird eine Lösung eingesetzt, die 40% a.TS an Saccharose und 0,32% a.TS an Caacetat enthält. Der pH-Wert der Lösung liegt zwischen 5,5 und 6,0.

Zusammensetzung:

**Tabelle 2:**

| Herstellung von Saccharidgemischen bei verschiedenen Temperaturen | | | | |
|---|---|---|---|---|
| % a.TS | 28°C | 50°C | 58°C | 62°C |
| Fructose | 3,8 | 8,4 | 8,4 | 3,6 |
| Glucose | 2,3 | 3,0 | 1,9 | 0,7 |
| Saccharose | 0,7 | 2,6 | 45,4 | 82,3 |
| Isomaltulose | 81,5 | 62,2 | 21,1 | 3,9 |
| Trehalulose | 9,1 | 10,9 | 4,5 | 0,9 |
| Isomaltose | 1,4 | 2,0 | 0,6 | < 0,1 |
| Isomelezitose | 0,8 | 10,6 | 18,0 | 8,6 |
| Trisaccharide | 0,4 | 0,4 | 0,1 | < 0,1 |

Es zeigt sich, daß bei Umlagerungen bei 50°C bis 62°C drastisch erhöhte Isomelezitose-Mengen gebildet werden.

### Beispiel 2:

### Stabilität des verwendeten Enzyms

Wie in Beispiel 1 beschrieben, wird ein mit verbrauchten immobilisierten Bakterienzellen (200 Einheiten/g TS (Trockensubstanz) Biokatalysator) gefüllter Säulenreaktor bei 50°C für 43 Tage betrieben.

**Tabelle 3:**

| Isomelezitose-Bildung bei 50°C | | |
|---|---|---|
| | Isomelezitose (% a.TS) | Fließrate |
| 1. Tag | 11,8 | 120 |
| 8. Tag | 10,9 | 123 |
| 15. Tag | 11,4 | 110 |
| 22. Tag | 11,0 | 114 |
| 29. Tag | 10,6 | 100 |
| 36. Tag | 10,3 | 95 |
| 43. Tag | 9,1 | 81 |

Die Ergebnisse der Tabelle 3 dokumentieren die hohe Stabilität des Enzyms bei den gewählten Bedingungen. Bei Verwendung eines nicht-verbrauchten frischen Biokatalysators (400 bis 450 Einheiten/g TS Biokatalysator) beträgt die Benutzungsdauer maximal 20 Tage.

### Beispiel 3:

### Herstellung von Isomelezitose-reicher Saccharidgemisch-Lösung

Die in Beispiel 2 erhaltene Lösung wird -wie in Beispiel 1 beschrieben- in einem mit immobilisierten Bakterienzellen gefüllten Säulenreaktor bei 25°C nochmals behandelt (Schritt 110, Figur), um eventuell noch vorhandene Saccharose praktisch vollständig zu Isomaltulose zu isomerisieren.

**Tabelle 4:**

| Zusammensetzung des Saccharidgemisches vor und nach nochmaliger enzymatischer Umlagerung | | |
|---|---|---|
| % a.TS | vorher | nachher |
| Fructose | 7,2 | 8,0 |
| Glucose | 2,5 | 3,0 |
| Saccharose | 34,4 | 0,3 |
| Isomaltulose | 37,2 | 64,5 |
| Trehalulose | 6,6 | 11,3 |
| Isomaltose | 1,2 | 1,5 |
| Isomelezitose | 10,5 | 11,0 |
| Trisaccharide | 0,2 | 0,3 |

### Beispiel 4:

### Herstellung von kristalliner Isomelezitose, Isomaltulose und hydrierter Isomaltulose

Ausgehend von der in Beispiel 3 anfallenden Lösung eines Saccharidgemisches (nach Schritt 110, Figur) mit einem Isomelezitose-Gehalt von 11,0% a.TS wird die Anreicherung der Isomelezitose und Isomaltulose bis zum kristallinen Produkt gestartet.
a) Erste Kühlungskristallisation (Entfernung und Gewinnung von Isomaltulose)
Die Lösung wird auf 81 % a.TS (11,4 kg Sirup) bei 80 bis 85°C konzentriert und anschließend unter langsamem Rühren einer Kühlungskristallisation mit einer Kühlungsrate von 1,5 K pro Stunde -bis eine Endtemperatur von 40 bis 45°C erreicht ist- unterzogen (Schritt 120, Figur). Die dabei gebildeten Kristalle werden mittels einer Siebkorbzentrifuge von der Mutterlauge getrennt und mit Wasser gewaschen. Die erhaltenen Kristalle sind weiß und bestehen zu 98,5% aus Isomaltulose. Die so erhaltene Isomaltulose kann mittels herkömmlicher Hydrierung, beispielsweise bei 60-120°C, vorzugsweise bei 80°C, mit Wasserstoffgas unter einem Druck von 8-12 MPa, vorzugsweise 10 MPa, mittels eines Raney-Nickel Katalysators, zu einem annähernd äquimolaren Gemisch aus 1,6-GPS und 1,1-GPM hydriert werden.
Die Abreicherung der Ausgangslösung an Isomaltulose beträgt 58%. Hingegen steigt der Gehalt an Isomelezitose auf 23,6% a.TS.
b) Chromatographie am Ca²⁺-beladenen Kationenaustauscher
Eine weitere Anreicherung der Isomelezitose erfolgt durch Chromatographie an einem stark sauren Kationenaustauscher in der Ca²⁺-Form (z.B. Amberlite XE 5 94) (Schritt 130, Figur).

| | |
|---|---|
| Trennanlage | 10 m Länge, Durchmesser 25 cm |
| Fließrate | 100 l/h, Elutionsmedium: entionisiertes Wasser, entgast |
| Aufgabelösung | 8,2 kg mit 50% a.TS |

Fraktionen mit Reinheiten an Isomelezitose ≥ 63% werden vereinigt und auf 60 % a.TS konzentriert.
c) Zweite Kühlungskristallisation
Eine zweite Kristallisation der Isomelezitose erfolgt -ausgehend von einer Lösung mit 60% a.TS- mittels Kühlungskristallisation (Schritt 140, Figur).

| | |
|---|---|
| Abkühlrate | 1,5°K pro Stunde |
| Starttemperatur | 55°C |
| Stop bei | 20°C |

Eine weitere Kühlungskristallisation kann sich anschließen:

| | |
|---|---|
| Lösung | 75% a.TS, sonstige Bedingungen wie zuvor |
| Ausbeute (insgesamt) | 0,54 kg (53%) |
| Reinheit | 98% |

Das erfindungsgemäße Verfahren führt also zur Darstellung reiner kristalliner Isomaltulose beziehungsweise hydrierter Isomaltulose und Isomelezitose.

### Beispiel 5:

### Fermentation mit Bäckerhefe

Das in Beispiel 3 anfallende Saccharidgemisch (40 kg, 40% a.TS) mit 8,0% a.TS an Fructose und 3% a.TS an Glucose wird mit entionisiertem Wasser auf 20% a.TS verdünnt. Nach erfolgter Zugabe der Bäckerhefe (15 g frische Hefe/kg Lösung) wird für 16 Stunden unter Rühren und Belüftung bei 35°C fermentiert (schritt 150, Figur).

Die Hefe wird anschließend durch Filtration beziehungsweise Zentrifugation abgetrennt.

Zusammensetzung:

**Tabelle 5:**

| Zusammensetzung des Saccharidgemisches nach Fermentation mit Bäckerhefe | |
|---|---|
| Kohlenhydrate | % a.TS |
| Isomaltulose | 73,0 |
| Trehalulose | 12,5 |
| Isomaltose | 1,7 |
| Isomelezitose | 12,5 |
| Trisaccharide | 0,2 |

Die Fermentation mit Bäckerhefe führt zum vollständigen Abbau von Glucose, Fructose und Saccharose.

### Beispiel 6:

### Hydrierung der Isomelezitose-reichen Saccharidgemisch-Lösung

Die nach Beispiel 3 hergestellte Isomelezitose-reiche Saccharidgemisch-Lösung wird an einem Raney-Nickel-Katalysator bei 60 bis 100°C mit Wasserstoffgas unter einem Druck bei 8 bis 12 MPa kontinuierlich hydriert (Schritt 160, Figur). Die erhaltene Lösung weist folgende Zusammensetzung auf:

**Tabelle 6:**

| Zusammensetzung des Saccharidgemisches nach Hydrierung | |
|---|---|
| | % a.TS |
| Sorbit | 7,8 |
| Mannit | 3,5 |
| 1,1-GPM | 34,5 |
| 1,1-GPS + 1,6-GPS | 41,4 |
| Isomelezitose | 11,3 |

Das erhaltene Gemisch stellt ein vorteilhaftes Süßungsmittel oder Futtermittel dar. Selbstverständlich kann aus dem aus der Umlagerung hervorgegangenen Saccharidgemisch Trehalulose und/oder Isomelezitose abgetrennt und das Rest-Saccharidgemisch hydriert werden. Das enstandene Süßungsmittelgemisch enthält dann 1,1-GM, 1,6-GPS and gegebenenfalls Sorbit, Mannit und 1,1-GPS.

### Beispiel 7:

### Hydrierung der Isomelezitose-reichen Saccharidgemisch-Lösung nach Behandlung mit Bäckerhefe

Vor der Hydrierung wird die Lösung mittels Kationen- und Anionenaustauscher entsalzt, entfärbt und von Nicht-Zuckerstoffen befreit.

Die Hydrierung erfolgt, wie in Beispiel 6 beschrieben. Die Lösung weist anschließend folgende Zusammensetzung auf:

**Tabelle 7:**

| Zusammensetzung des Saccharidgemisches nach Hydrierung und Bäckerhefe-Behandlung | |
|---|---|
| | % a.TS |
| Sorbit | 0,1 |
| Mannit | < 0,1 |
| 1,1-GPM | 37,8 |
| 1,1-GPS + 1,6-GPS | 47,5 |
| Isomelezitose | 13,2 |

Das erhaltene Gemisch, das im Vergleich zum Süßungsmittel in Beispiel 6, kaum Mannit und Sorbit enthält, stellt ein vorzügliches Süßungs- und Futtermittel dar. Auch bei der Herstellung dieses Gemisches kann vorgesehen sein, Isomelezitose und/oder Trehalulose vor der Hydrierung aus dem zu hydrierenden Saccharidgemisch zu entfernen.

### Beispiel 8:

### Stabilität der Isomelezitose bei pH 4 und 95°C

Jeweils 1%-ige Lösungen von Isomelezitose beziehungsweise Saccharose werden mit 50 mM Na-Acetat-Puffer, pH 4,0, hergestellt und anschließend im Eppendorf-Heizblock bei 95°C inkubiert. Proben werden zu verschiedenen Zeiten genommen und analysiert.

Ergebnis:

**Tabelle 8:**

| Stabilität der Isomelezitose | | | | |
|---|---|---|---|---|
| | Zeit [Min] | | | |
| % Hydrolyse | 0 | 60 | 120 | 180 |
| Saccharose | 0 | 13 | 24 | 37 |
| Isomelezitose | 0 | 7 | 13 | 19 |

Isomelezitose ist nicht so Hydrolyse-anfällig wie Saccharose.

### Beispiel 9:

### Enzymatische Spaltung mit Invertase (Hefe)

Jeweils 1%-ige Lösungen von Isomelezitose beziehungsweise Saccharose werden in 50 mM Na-Acetat-Puffer, pH 5,4, mit 0,4 Einheiten Invertase pro ml bei 37°C für 22 Stunden inkubiert.

Ergebnis:

**Tabelle 9:**

| Invertase-Spaltung | | | | | |
|---|---|---|---|---|---|
| | Zeit [Std.] | | | | |
| % Hydrolyse | 0 | 1 | 2 | 4 | 22 |
| Saccharose | 0 | 34 | 50 | 81 | 100 |
| Isomelezitose | 0 | < 1 | < 1 | < 1 | < 1 |

Hefeinvertase spaltet Isomelezitose nicht.

### Beispiel 10:

### Spaltungsversuch von Isomelezitose mit Saccharase/ Isomaltase- und Glucoamylase/Maltase-Enzymkomplexen der Dünndarm-Mucosa (Schwein)

Die aus der Dünndarm-Mucosa des Schweins isolierten Enzymkomplexe (Saccharase/Isomaltase (S/I) und Glucoamylase/Maltase (G/M)) sind mit Isomelezitose beziehungsweise Maltose inkubiert worden. Jeweils 20 mM Isomelezitose beziehungsweise Maltose werden dazu in 0,1 M Triethanolamin-Hydrochlorid-Puffer, pH = 7,0, mit jeweils 0,7 Einheiten Enzymkomplex (Aktivität bestimmt mit Maltose als Substrat) bei 37°C inkubiert. Die Reaktion wird durch Erhitzen auf 94°C für 3Minuten gestoppt.

Ergebnis:

**Tabelle 10:**

| Enzymatische Spaltungen | | |
|---|---|---|
| | % Hydrolyse | |
| | G/M | S/I |
| Maltose | 100 | 96 |
| Isomelezitose | 0 | 3 |

Isomelezitose wird praktisch nicht gespalten.

### Beispiel 11 :

### Umlagerung, Hydrierung, Trocknung; Schritte 100, 160, 170

Die enzymatische Umlagerung (Schritt 100) der Saccharose bei 50°C ergibt folgende Zusammensetzung der Produktlösung (als % der umgesetzten Saccharose):

**Tabelle 11**

| | % a.TS |
|---|---|
| Fructose | 8,6 |
| Glucose | 3,1 |
| Isomaltulose | 63,9 |
| Trehalulose | 11,2 |
| Isomaltose | 2,1 |
| Isomelezitose | 10,9 |
| andere Trisaccharide | 0,4 |

Die Hydrierung (Schritt 160) dieser Lösung ergibt eine Lösung folgender Zusammensetzung (% a.TS):

**Tabelle 12**

| | % a.TS |
|---|---|
| Mannit | 4,3 |
| Sorbit | 7,4 |
| 1,1 GPM | 39,8 |
| 1,1-GPS | 3,4 |
| 1,6-GPS | 34,0 |
| Isomelezitose | 10,9 |
| andere Trisaccharide | 0,4 |

Die Trocknung (Schritt 170) dieses Produkts führt zum gewünschten Süßungsmittel.

### Beispiel 12:

### Umlagerung, Vergärung mit Hefe, Hydrierung, Trocknung; Schritte 100, 150, 160, 170

Die enzymatische Umlagerung (Schritt 100) der Saccharose bei 50°C ergibt die unter Beispiel 11 angegebene Zusammensetzung.

Die Vergärung mit Hefe (Schritt 150) entfernt Fructose und Glucose, wobei sich folgende Zusammensetzung (% a.TS) der Produktlösung ergibt:

**Tabelle 13**

| | % a.TS |
|---|---|
| Isomaltulose | 72,2 |
| Trehalulose | 12,6 |
| Isomaltose | 2,4 |
| Isomelezitose | 12,3 |
| andere Trisaccharide | 0,5 |

Die Hydrierung dieser Lösung (Schritt 160) führt zu folgendem Produkt:

**Tabelle 14**

| | % a.TS |
|---|---|
| 1,1-GPM | 44,9 |
| 1,1-GPS | 3,8 |
| 1,6-GPS | 38,5 |
| Isomelezitose | 12,3 |
| andere Trisaccharide | 0,5 |

### Beispiel 13:

### Umlagerung, Kristallisation eines Teils der Isomaltulose, Hydrierung der Mutterlauge, Trocknung; Schritte 100, 120, 160, 170

Die enzymatische Umlagerung (Schritt 100) der Saccharose bei 50°C ergibt die unter Beispiel 11 angegebene Zusammensetzung.

Die Kristallisation (Schritt 120), zum Beispiel 50%, der gebildeten Isomaltulose, führt zu einer Mutterlauge mit folgender Zusammensetzung:

**Tabelle 15**

| | % a.TS |
|---|---|
| Fructose | 12,6 |
| Glucose | 4,5 |
| Isomaltulose | 46,8 |
| Trehalulose | 16,4 |
| Isomaltose | 3,1 |
| Isomelezitose | 15,9 |
| andere Trisaccharide | 0,6 |

Durch Hydrierung (Schritt 160) dieser Mutterlauge erhält man folgendes Produkt:

**Tabelle 16**

| | % a.TS |
|---|---|
| Mannit | 6,3 |
| Sorbit | 10,8 |
| 1,1-GPM | 34,9 |
| 1,1-GPS | 4,9 |
| 1,6-GPS | 26,5 |
| Isomelezitose | 15,9 |
| andere Trisaccharide | 0,6 |

Die Trocknung (Schritt 170) dieser Lösung führt zum gewünschten Süßungsmittel.

Die bei dieser Verfahrensvariante als Nebenprodukt erhaltene Isomaltulose kann als solches oder nach Hydrierung als Süßungsmittel Verwendung finden.

### Beispiel 14:

### Umlagerung, Vergärung mit Hefe, Kristallisation eines Teils der Isomaltulose, Hydrierung der Mutterlauge, Trocknung; Schritte 100, 150, 120, 160

Die enzymatische Umlagerung der Saccharose bei 50°C ergibt die unter Beispiel 11 angegebene Zusammensetzung.

Die Vergärung mit Hefe (Schritt 150) entfernt Fructose und Glucose, wobei sich folgende Zusammensetzung der Produktlösung ergibt:

**Tabelle 17**

| | % a.TS. |
|---|---|
| Isomaltulose | 72,2 |
| Trehalulose | 12,6 |
| Isomaltose | 2,4 |
| Isomelezitose | 12,3 |
| andere Trisaccharide | 0,5 |

Die Kristallisation (Schritt 120) von zum Beispiel 70% der vorliegenden Isomaltulose führt zu einer Mutterlauge mit folgender Zusammensetzung:

**Tabelle 18**

| | % a.TS |
|---|---|
| Isomaltulose | 43,8 |
| Trehalulose | 25,5 |
| Isomaltose | 4,8 |
| Isomelezitose | 24,8 |
| andere Trisaccharide | 1,0 |

Durch die Hydrierung (Schritt 160) dieser Lösung erhält man eine Produktlösung mit folgender Zusammensetzung:

**Tabelle 19**

| | % a.TS |
|---|---|
| 1,1-GPM | 39,7 |
| 1,1-GPS | 7,7 |
| 1,6-GPS | 26,7 |
| Isomelezitose | 24,8 |
| andere Trisaccharide | 1,0 |

Die Trocknung (Schritt 170) dieses Produkts führt zum gewünschten Süßungsmittel.

### Beispiel 15:

### Umlagerung, Vergärung mit Hefe, Kristallisation eines Teils der Isomaltulose, chromatographische Trennung, Hydrierung, Trocknung; Schritte 100, 150, 120, 130, 160, 170

Die enzymatische Umlagerung (Schritt 100) und Vergärung mit Hefe (Schritt 150) sowie Kristallisation der Isomaltulose (Schritt 120) sind identisch mit den entsprechenden Verfahrensschritten in Beispiel 14, so daß die nach der Kristallisation erhaltene Mutterlauge die in Tabelle 18 aufgeführte Zusammensetzung aufweist.

Die chromatographische Trennung (Schritt 130) dieser Mutterlauge führt zu einer mit Isomelezitose angereicherten Fraktion mit folgender Zusammensetzung:

**Tabelle 20**

| | % a.TS |
|---|---|
| Isomaltulose | 22,7 |
| Trehalulose | 5,7 |
| Isomaltose | 7,5 |
| Isomelezitose | 63,7 |
| andere Trisaccharide | 0,4 |

Durch die Hydrierung (Schritt 160) dieser Lösung erhält man eine Produktlösung mit folgender Zusammensetzung:

**Tabelle 21**

| | % a.TS |
|---|---|
| 1,1-GPM | 15,4 |
| 1,1-GPS | 1,7 |
| 1,6-GPS | 18,8 |
| Isomelezitose | 63,7 |
| andere Trisaccharide | 0,4 |

Die Trocknung (Schritt 170) dieses Produkts führt zum gewünschten Süßungsmittel.

### Beispiel 16:

### Umlagerung, Vergärung mit Hefe, Kristallisation eines Teils der Isomaltulose, chromatographische Trennung, Kristallisation der Isomelezitose; Schritte 100, 150, 120, 130, 140

Die Teilschritte enzymatische Umlagerung (Schritt 100), Vergärung mit Hefe (Schritt 150), Kristallisation eines Teils der Isomaltulose (Schritt 120) und chromatographische Trennung (Schritt 130) sind identisch mit den entsprechenden Verfahrensschritten des Beispiels 15, so daß die nach der chromatographischen Trennung erhaltene, Isomelezitose-reiche Fraktion die in Tabelle 20 aufgeführte Zusammensetzung aufweist.

Kristallisation: Aus der Isomelezitose-reichen Fraktion läßt sich die Isomelezitose durch Verdampfung und Kühlung auskristallisieren (Schritt 140).

Isomelezitosekristalle lassen sich an einer Siebkorb-Zentrifuge von der Mutterlauge abtrennen und mit Wasser waschen.

Die Trocknung dieses Produkts führt zum gewünschten Süßungsmittel.

## Patentansprüche

1. Verfahren zur Herstellung eines Süßungsmittels, wobei man in einem ersten Verfahrensschritt enzymatisch Saccharose unter Verwendung von Zellen von Protaminobacter rubrum oder der Saccharose-6-glucosylmutase bei einer Temperatur von 50 bis 60°C umwandelt und in einem zweiten Verfahrensschritt das erhaltene Saccharidgemisch katalytisch hydriert.

2. Verfahren nach Anspruch 1, **dadurch gekennzeichnet, dass** das erhaltene Saccharidgemisch einen Isomelezitose-Gehalt von mehr als 10 Gew.-% (a.TS) aufweist.

3. Verfahren zur Herstellung von Isomelezitose und Isomaltulose, wobei man eine Saccharoselösung enzymatisch unter Verwendung von Zellen von Protaminobacter rubrum oder der Saccharose-6-glucosylmutase in eine Lösung eines Saccharidgemisches bei einer Temperatur von 50 bis 60° umwandelt, aus der so erhaltenen Lösung eines Saccharidgemisches die gebildete Isomaltulose durch Kristallisation weitgehend entfernt und dabei die Isomelezitose in einer Mutterlauge anreichert, die Mutterlauge einer chromatographischen Trennung unterzieht und dabei aus dem Eluat die an Isomelezitose angereicherten Fraktionen sammelt und diese zu einer Isomelezitosefraktion vereinigt, konzentriert, die dabei entstandenen Isomelezitose-Kristalle von der Mutterlauge abtrennt, wäscht und die Isomelezitose in reiner kristalliner Form erhält.

4. Verfahren nach Anspruch 3, **dadurch gekennzeichnet, dass** aus der erhaltenen Lösung eines Saccharidgemisches die gebildete Isomaltulose durch Verdampfungs- und Kühlungskristallisation weitgehend entfernt wird.

5. Verfahren nach Anspruch 3 oder 4, **dadurch gekennzeichnet, dass** die Isomelezitosefraktion durch Eindampfen und Kühlungskristallisation konzentriert wird.

6. Verfahren nach Anspruch 3, 4 oder 5, **dadurch gekennzeichnet, dass** die Abtrennung der Isomelezitose-Kristalle von der Mutterlauge durch Zentrifugation geschieht.

7. Verfahren nach einem der vorhergehenden -Ansprüche, **dadurch gekennzeichnet, dass** im Anschluss an die enzymatische Umwandlung bei 50 bis 60°C und vor der gegebenenfalls stattfindenden katalytischen Hydrierung eine zweite, enzymatische Umlagerung bei 25 bis 30°C durchgeführt wird.

8. Verfahren nach einem der vorhergehenden Ansprüche, **dadurch gekennzeichnet, dass** die erste und die gegebenenfalls stattfindende zweite enzymatische Umwandlung mittels Zellen von Protaminobacter rubrum (CBS 574.77) durchgeführt wird.

9. Verfahren nach Anspruch 8, **dadurch gekennzeichnet, dass** die Zellen von Protaminobacter rubrum immobilisiert sind.

10. Verfahren nach einem der vorhergehenden Ansprüche, **dadurch gekennzeichnet, dass** die Zellen von Protaminobacter rubrum gebraucht sind.

11. Verfahren nach einem der vorhergehenden Ansprüche, **dadurch gekennzeichnet, dass** die erste und die gegebenenfalls stattfindende zweite enzymatische Umwandlung mittels der Saccharose-6-glucosylmutase (EC 5.4.99.10) durchgeführt wird.

12. Verfahren nach einem der vorhergehenden Ansprüche, **dadurch gekennzeichnet, dass** die erste enzymatische Umlagerung kontinuierlich über einen Zeitraum von 40 bis 50 Tagen durchgeführt wird.

13. Verfahren nach einem der vorhergehenden Ansprüche, **dadurch gekennzeichnet, dass** im Anschluss an die erste oder die gegebenenfalls stattfindende zweite enzymatische Umlagerung und vor der katalytischen Hydrierung das erhaltene Saccharidgemisch mit freien oder immobilisierten Zellen von Bäckerhefe für einen Zeitraum in Kontakt gebracht wird, der dazu ausreicht, eventuell vorhandene Monosaccharide zu vergären.

14. Verfahren nach einem der vorhergehenden Ansprüche, **dadurch gekennzeichnet, dass** das erhaltene Saccharidgemisch vor der katalytischen Hydrierung von nicht umgewandelter Rest-Saccharose durch enzymatische oder H⁺-katalytische Spaltung befreit wird.

15. Verfahren nach einem der vorhergehenden Ansprüche, **dadurch gekennzeichnet, dass** entweder vor oder nach der katalytischen Hydrierung das erhaltene Saccharidgemisch einer chromatographischen Trennung unterworfen wird.

16. Verfahren nach einem der vorhergehenden Ansprüche, **dadurch gekennzeichnet, dass** die chromatographische Trennung an mit Calcium beladenen stark sauren, schwach vernetzten Kationenaustauscherharzen durchgeführt wird.

17. Verfahren nach einem der vorhergehenden Ansprüche, **dadurch gekennzeichnet, dass** man nach der enzymatischen Umwandlung aus dem erhaltenen Saccharidgemisch Isomaltulose abtrennt und die Isomaltulose katalytisch hydriert.

18. Verfahren nach einem der vorhergehenden Ansprüche, **dadurch gekennzeichnet, dass** man nach der enzymatischen Umwandlung aus dem erhaltenen Saccharidgemisch Trehalulose und/oder Isomelezitose abtrennt und das verbleibende Saccharidengemisch katalytisch hydriert.

19. Verfahren nach einem der vorhergehenden Ansprüche, **dadurch gekennzeichnet, dass** man ein Süßungsmittelgemisch aus Isomelezitose, 1,1-GPM (1-0-α-D-Glucopyranosyl-D-mannit), und 1,6-GPS (6-O-α-D-Glucopyranosyl-D-sorbit), sowie gegebenenfalls 1,1-GPS (1-O-α-D-Glucopyranosyl-D-sorbit), Mannit und Sorbit erhält.

20. Verfahren nach einem der vorhergehenden Ansprüche, **dadurch gekennzeichnet, dass** man ein Süßungsmittelgemisch aus 1,1-GPM (1-O-α-D-Glucopyranosyl-D-mannit), und 1,6-GPS (6-O-α-D-Glucopyranosyl-D-sorbit), sowie gegebenenfalls 1,1-GPS (1-O-α-D-Glucopyranosyl-D-sorbit), Mannit und Sorbit erhält.

21. Verfahren nach einem der vorhergehenden Ansprüche, **dadurch gekennzeichnet, dass** im Anschluss an die erste oder die gegebenenfalls stattfindende zweite enzymatische Umlagerung sowie vor oder nach dem gegebenenfalls stattfindenden Inkontaktbringen des erhaltenen Saccharidgemisches mit Bäckerhefe die bei der enzymatischen Umlagerung gebildete Isomaltulose durch Kristallisation, insbesondere Verdampfungs- und Kühlungskristallisation, entfernt wird.

22. Süßungsmittelgemisch, **dadurch gekennzeichnet, dass** dieses 1,1-GPM (1-O-α-D-Glucopyranosyl-D-mannit), 1,6-GPS (6-O-α-D-Glucopyranosyl-D-sorbit), mehr als 10 Gew.-% Isomelezitose und gegebenenfalls Sorbit, Mannit und 1,1-GPS (1-O-α-D-Glucopyranosyl-D-sorbit) enthält.

23. Süßungsmittelgemisch nach Anspruch 22, wobei dieses 10 bis 15 Gew.-%, 20 bis 30 Gew.-% oder 60 bis 80 Gew.-% (bezogen auf Trockensubstanz) Isomelezitose enthält.

24. Süßungsmittelgemisch nach Anspruch 22 oder 23, wobei diese 1,1-GPM (1-O-α-D-Glucopyranosyl-D-mannit) und 1,6-GPS (6-O-α-D-Glucopyranosyl-D-sorbit) in äquimolaren Mengen enthält.

## Claims

1. Process for producing a sweetener in which, in a first process step, sucrose is enzymatically converted using cells of Protaminobacter rubrum or sucrose-6-glucosylmutase at a temperature of 50 to 60°C and, in a second process step, the resultant saccharide mixture is catalytically hydrogenated.

2. Process according to Claim 1, **characterized in that** the resultant saccharide mixture has an isomelezitose content greater than 10% by weight (DM).

3. Process for producing isomelezitose and isomaltulose, in which a sucrose solution is enzymatically converted into a solution of a saccharide mixture using cells of Protaminobacter rubrum or sucrose-6-glucosylmutase at a temperature of 50 to 60°, from the resultant saccharide mixture solution the isomaltulose formed is substantially removed by crystallization, and the isomelezitose is enriched in a mother liquor, the mother liquor is subjected to a chromatographic separation and from the eluate the isomelezitose-enriched fractions are collected and these are combined to form an isomelezitose fraction, the fraction is concentrated, the resultant isomelezitose crystals are separated off from the mother liquor, washed and the isomelezitose is obtained in a pure crystalline form.

4. Process according to Claim 3, **characterized in that**, from the resultant saccharide mixture solution, the isomaltulose formed is substantially removed by evaporative crystallization and cooling crystallization.

5. Process according to Claim 3 or 4, **characterized in that** the isomelezitose fraction is concentrated by evaporation and cooling crystallization.

6. Process according to Claim 3, 4 or 5, **characterized in that** the isomelezitose crystals are separated off from the mother liquor by centrifugation.

7. Process according to one of the preceding claims, **characterized in that**, after the enzymatic conversion at 50 to 60°C, and before any catalytic hydrogenation taking place, a second enzymatic rearrangement is carried out at 25 to 30°C.

8. Process according to one of the preceding claims, **characterized in that** the first enzymatic conversion, and where it takes place the second enzymatic conversion, are carried out by means of cells of Protaminobacter rubrum (CBS 574.77).

9. Process according to Claim 8, **characterized in that** the cells of Protaminobacter rubrum are immobilized.

10. Process according to one of the preceding claims, **characterized in that** the cells of Protaminobacter rubrum have been previously used.

11. Process according to one of the preceding claims, **characterized in that** the first enzymatic conversion, and where it takes place the second enzymatic conversion, are carried out using sucrose-6-glucosylmutase (EC 5.4.99.10).

12. Process according to one of the preceding claims, **characterized in that** the first enzymatic rearrangement is carried out continuously over a period of 40 to 50 days.

13. Process according to one of the preceding claims, **characterized in that**, after the first enzymatic rearrangement, or where it takes place the second enzymatic rearrangement, and before the catalytic hydrogenation, the resultant saccharide mixture is brought into contact with free or immobilized cells of baker's yeast for a period which is sufficient to ferment any monosaccharides present.

14. Process according to one of the preceding claims, **characterized in that** the resultant saccharide mixture, before the catalytic hydrogenation, is freed from unconverted residual sucrose by enzymatic or H⁺-catalysed cleavage.

15. Process according to one of the preceding claims, **characterized in that** either before or after the catalytic hydrogenation, the resultant saccharide mixture is subjected to a chromatographic separation.

16. Process according to one of the preceding claims, **characterized in that** the chromatographic separation is carried out on calcium-loaded, highly acidic, weakly crosslinked cation exchange resins.

17. Process according to one of the preceding claims, **characterized in that**, after the enzymatic conversion, isomaltulose is separated off from the resultant saccharide mixture and the isomaltulose is catalytically hydrogenated.

18. Process according to one of the preceding claims, **characterized in that**, after the enzymatic conversion, trehalulose and/or isomelezitose is separated off from the resultant saccharide mixture and the remaining saccharide mixture is catalytically hydrogenated.

19. Process according to one of the preceding claims, **characterized in that** a sweetener mixture of isomelezitose, 1,1-GPM (1-O-α-D-glucopyranosyl-D-mannitol), and 1,6-GPS (6-O-α-D-glucopyranosyl-D-sorbitol), and if appropriate 1,1-GPS (1-O-α-D-glucopyranosyl-D-sorbitol), mannitol and sorbitol is obtained.

20. Process according to one of the preceding claims, **characterized in that** a sweetener mixture of 1,1-GPM (1-O-α-D-glucopyranosyl-D-mannitol), and 1,6-GPS (6-O-α-D-glucopyranosyl-D-sorbitol), and if appropriate 1,1-GPS (1-O-α-D-glucopyranosyl-D-sorbitol), mannitol and sorbitol is obtained.

21. Process according to one of the preceding claims, **characterized in that**, after the first enzymatic rearrangement, or where it takes place the second enzymatic rearrangement, and before or after the contacting of the resultant saccharide mixture, where this occurs, with baker's yeast, the isomaltulose formed in the enzymatic rearrangement is removed by crystallization, in particular evaporative crystallization and cooling crystallization.

22. Sweetener mixture, **characterized in that** this comprises 1,1-GPM (1-O-α-D-glucopyranosyl-D-mannitol), 1,6-GPS (6-O-α-D-glucopyranosyl-D-sorbitol), more than 10% by weight of isomelezitose and, if appropriate, sorbitol, mannitol and 1,1-GPS (1-O-α-D-glucopyranosyl-D-sorbitol).

23. Sweetener mixture according to Claim 22, where this comprises 10 to 15% by weight, 20 to 30% by weight, or 60 to 80% by weight (based on dry matter), of isomelezitose.

24. Sweetener mixture according to Claim 22 or 23, where this comprises 1,1-GPM (1-O-α-D-glucopyranosyl-D-mannitol) and 1,6-GPS (6-O-α-D-glucopyranosyl-D-sorbitol) in equimolar amounts.

## Revendications

1. Procédé de fabrication d'un agent édulcorant selon lequel, dans une première étape, on transforme du saccharose par voie enzymatique en utilisant des cellules de protaminobacter rubrum ou de s accharose-6-glucosylmutase à une température de 50 à 60°C et
dans une seconde étape on hydrate catalytiquement le mélange de saccharides obtenu.

2. Procédé selon la revendication 1,
**caractérisé en ce que**
le mélange de saccharides obtenu a une teneur en isomélézitose supérieure à 10 % en poids (matières sèches).

3. Procédé de fabrication d'isomélézitose et d'isomaltulose selon lequel
on transforme une solution de saccharose par voie enzymatique en utilisant des cellules de protaminobacter rubrum ou de saccharose-6-glucosylmutase dans une solution d'un mélange de saccharides à une température comprise entre 50 et 60°C,
on élimine largement l'isomaltulose formée de la solution d'un mélange de saccharides obtenue en procédant par cristallisation et on enrichit ainsi la lessive mère en isomélézitose,
on soumet cette lessive mère à une séparation par chromatographie et
à partir du produit élué on collecte les fractions enrichies en isomélézitose et on les réunit en une fraction d'isomélézitose, que l'on concentre, on sépare les cristaux d'isomélézitose obtenus de la lessive mère, on les lave et on obtient l'isomélézitose sous forme cristalline pure.

4. Procédé selon la revendication 3,
**caractérisé en ce qu'**
à partir de la solution d'un mélange de saccharides obtenue, on élimine largement l'isomaltulose formée par évaporation et en cristallisation par refroidissement.

5. Procédé selon l'une quelconque des revendications 3 ou 4,
**caractérisé en ce qu'**
on concentre la fraction d'isomélézitose par évaporation et cristallisation par refroidissement.

6. Procédé selon l'une quelconque des revendications 3, 4, 5,
**caractérisé en ce qu'**
on sépare les cristaux d'isomélézitose de la lessive mère en procédant par centrifugation.

7. Procédé selon l'une quelconque des revendications précédentes,
**caractérisé en ce qu'**
après la transformation enzymatique à 50-60°C et le cas échéant avant l'hydratation catalytique, on effectue une seconde transformation enzymatique à une température comprise entre 25°C et 30°C.

8. Procédé selon l'une quelconque des revendications précédentes,
**caractérisé en ce qu'**
on effectue la première et l'éventuelle seconde transformation enzymatiques à l'aide de cellules de protaminobacter rubrum (CBS 574.77).

9. Procédé selon la revendication 8,
**caractérisé en ce qu'**
on immobilise les cellules de protaminobacter rubrum.

10. Procédé selon l'une quelconque des revendications précédentes,
**caractérisé en ce que**
les cellules de protaminobacter rubrum ont été utilisées antérieurement.

11. Procédé selon l'une quelconque des revendications précédentes,
**caractérisé en ce qu'**
on effectue la première et l'éventuelle seconde transformation enzymatiques à l'aide de saccharose-6-glucolsylmutase (EC 5.4.99.10).

12. Procédé selon l'une quelconque des revendications précédentes,
**caractérisé en ce qu'**
on effectue la première transformation enzymatique en continu dans une période de 40 à 50 jours.

13. Procédé selon l'une quelconque des revendications précédentes,
**caractérisé en ce qu'**
à la suite de la première et de l'éventuelle seconde transformation enzymatique et avant l'hydratation catalytique, on met le mélange de saccharides, obtenu, en contact avec des cellules libres ou immobilisées de levure boulangère, pendant une durée suffisante pour fermenter les monosaccharides éventuellement présentes.

14. Procédé selon l'une quelconque des revendications précédentes,
**caractérisé en ce qu'**
avant l'hydratation catalytique on débarrasse le mélanges de saccharides obtenu du saccharose résiduel non transformé par dissociation par voie enzymatique ou catalytique par les ions H⁺.

15. Procédé selon l'une quelconque des revendications précédentes,
**caractérisé en ce que**
soit avant soit après l'hydratation catalytique on soumet le mélange de saccharides, obtenu, à une séparation par chromatographie.

16. Procédé selon l'une quelconque des revendications précédentes,
**caractérisé en ce qu'**
on effectue la séparation par chromatographie sur des résines échangeuses de cations, faiblement réticulées, fortement acides, chargées de calcium.

17. Procédé selon l'une quelconque des revendications précédentes,
**caractérisé en ce qu'**
après la transformation par voie enzymatique, on sépare l'isomaltulose du mélange de saccharides obtenu, et on l'hydrate par voie catalytique.

18. Procédé selon l'une quelconque des revendications précédentes,
**caractérisé en ce qu'**
après la transformation par voie enzymatique, on sépare la tréhalulose et/ou l'isomélézitose du mélange de saccharides obtenu, et on hydrate le mélange de saccharides résiduel par voie catalytique.

19. Procédé selon l'une quelconque des revendications précédentes,
**caractérisé en ce qu'**
on obtient un mélange d'agents édulcorant renfermant de l'isomélézitose, du 1,1-GPM (1-0-□-D-glucopyranosyl-D-mannitol) et du 1,6-GPS (6-0-□-D-glucopyranosyl-D-sorbitol) et le cas échéant du 1,1-GPS (1-0-□-D-glucopyranosyl-D-sorbitol), du mannitol et du sorbitol.

20. Procédé selon l'une quelconque des revendications précédentes,
**caractérisé en ce qu'**
on obtient un mélange d'agents édulcorant renfermant du, 1,1-GPM (1-0-□-D-glucopyranosyl-D-mannitol) et du 1,6-GPS (6-0-□-D-glucopyranosyl-D-sorbitol) et le cas échéant du 1,1-GPS (1-0-□-D-glucopyranosyl-D-sorbitol), du mannitol et du sorbitol.

21. Procédé selon l'une quelconque des revendications précédentes,
**caractérisé en ce qu'**
à la suite de la première et le cas échéant de la seconde transformation enzymatique et avant ou après l'éventuelle mise en contact du mélange de saccharides obtenu avec de la levure boulangère, on élimine l'isomaltulose formée par la transformation par voie enzymatique par cristallisation, notamment par évaporation et par refroidissement.

22. Mélange d'agents édulcorants,
**caractérisé en ce qu'**
il contient 1,1-GPM (1-0-□-D-glucopyranosyl-D-mannitol) et 1,6-GPS (6-0-□-D-glucopyranosyl-D-sorbitol) et le cas échéant du 1,1-GPS (1-0-□-D-glucopyranosyl-D-sorbitol), du mannitol et du sorbitol, du 1,1-GPM (1-0-□-D-Glucopyranosyl-D-mannitol) du 1,6-GPS (6-0-□-D-Glucopy-ranosyl-D-sorbitol), plus de 10 % en poids d'isomélézitose et le cas échéant du sorbitol, du mannitol et du 1,1-GPS (1-0-□-D-Glucopyranosyl-D-sorbitol).

23. Mélange d'agents édulcorants selon la revendication 22,
**caractérisé en ce qu'**
il contient entre 10 et 15 % en poids, 20 à 30 % en poids ou 60 à 80 % en poids (rapportés aux produits secs) d'isomélézitose.

24. Mélange d'agents édulcorants selon les revendication 22 ou 23,
**caractérisé en ce qu'**
il contient en quantité équimolaire du 1,1-GPM (1-0-□-D-glucopyranosyl-D-mannitol) et du 1,6-GPS (6-0-□-D-glucopyranosyl-D-sorbitol).
